# EUROPEAN PATENT APPLICATION

(11) **EP 3 842 033 A1**
(43) Date of publication of application: **30.06.2021**
(21) Application number: 20203959.0
(22) Date of filing: 06.07.2017
(51) Int. Cl.: A61K 9/14, A61K 31/047, A61K 31/765

(54) **INOSITOL COMPOSITIONS AND METHODS FOR USING SAME**

(30) Priority: 06.07.2016 US 201662358948 P
(62) Divisional of application: 17736666.3
(71) Applicant: Nogra Pharma Limited, Dublin 2 (IE)
(72) Inventor: BELLINVIA, Salvatore, 6850 Mendrisio (CH); DEMARTIS, Salvatore, 20159 Milan (IT); LARSSON, Helena, Dublin 2 D02 VY52 (IE); MCNULTY, Marie, Dublin 2 D02 VY52 (IE); VITI, Francesca, 6872 Salorino (CH)
(74) Representative: Lau, Sarah Jane

(57) **Abstract**

Disclosed herein are compositions comprising polyethylene glycol and inositol, which compositions can be useful for treating constipation. Also disclosed are methods of making and using the compositions.

## Description

### CROSS-REFERENCE TO RELATED APPLICATIONS

This application claims the benefit of and priority to U.S. Patent Application No. 62/358,948, filed July 6, 2016, the entire contents of which are incorporated by reference herein.

### BACKGROUND

Constipation, a common gastrointestinal symptom, is a delay in the discharge of dry and hard feces. In the general population, the rate of constipation varies from 2 to 30 percent. Among elderly people living in care homes, the rate of constipation is about 50-75 percent. In children, the prevalence of constipation ranges from 0.7% to 29.6%. In the United States, expenditures on medications for constipation are greater than US $250 million per year.

Constipation can result from many causes, for example, obstructed defecation and colonic slow transit (or hypomobility). A majority of patients evaluated for constipation may have obstructed defecation. This type of constipation has mechanical and functional causes. Causes of colonic slow transit constipation include diet, hormonal disorders such as hypothyroidism, side effects of medications, and rare heavy metal toxicity. Because constipation is a symptom, not a disease, effective treatment of constipation may require first determining the cause.

Certain medications, such as opioids, including codeine phosphate and morphine, are widely used as analgesics and are known to cause constipation as a troublesome and often serious complication. The effect is particularly troublesome among in-patients requiring prolonged opioid therapy in high doses such as terminally ill patients with a malignant disease.

Constipation patients suffer from symptoms such as a sensation of pressure, flatulence, and pains during the discharge of feces. Severe constipation includes obstipation (failure to pass stools or gas) and fecal impaction, which can progress to bowel obstruction and become life-threatening. Constipation is commonly treated with laxatives which help to soften the hard feces and promote defecation.

Macrogol (polyethylene glycol) is used as an osmotic laxative for the treatment of constipation in both adults and children. Macrogols are identified by a number which represents their molecular weight (e.g., macrogol 4000, macrogol 3350, macrogol 6000). Osmotic laxatives cause water to be retained with the stool, thereby increasing the number of bowel movements and softening the stool for easier passage. Since osmotic laxatives can take more than 24-48 hrs to have any effect, there is a need to develop a formulation suitable for the rapid relief of constipation.

### SUMMARY

Provided herein is a dry composition for the treatment of constipation where the dry composition generally includes: polyethylene glycol, inositol, and one or more pharmaceutically acceptable excipients. When administered to a patient, a disclosed composition may be effective in relieving constipation in a patient. For example, such relief can be in about half the time or less as compared to administering a composition having polyethylene glycol and no inositol. Such compositions for use in therapy and, more specifically, for use in the treatment of constipation are also provided. Furthermore, the use of such compositions in the manufacture of medicaments for use in the treatment of constipation is also provided herein.

Compositions may comprise about 90-98% (e.g., about 95-98%, about 96%, about 98%) by weight polyethylene glycol. Compositions may comprise between about 0.01% to about 2% (e.g., about 0.5%) by weight inositol. Pharmaceutically acceptable excipients may include soy lecithin, citric acid, and a flavor and/or a flavourant. Also provided herein, in some embodiments, is a liquid composition for oral administration. Liquid compositions may include a liquid carrier and a dry composition as disclosed herein, for example, a dry composition as disclosed herein mixed with water.

For example, provided herein, is a unit dosage form for the treatment of constipation comprising: polyethylene glycol, inositol, and one or more pharmaceutically acceptable excipients.

Also provided herein is a method of treating constipation, where the method includes administering to a patient in need thereof, a composition or a unit dosage disclosed herein. For example, provided herein is a method of treating constipation, wherein the method comprises dissolving a disclosed dry composition in water; and administering the final dissolved composition orally to a patient in need thereof.

A kit for the treatment of constipation also is provided, where the kit can include a composition or a unit dosage disclosed herein; and instructions for use in treating the disorder. A kit may further comprise a container. For example, a kit may comprise 2 sachets that include about 10 g of polyethylene glycol and between about 1 mg to about 60 mg of inositol, and instructions indicating that the 2 sachets are suitable for treating an adult patient and 1 sachet is suitable for treating a pediatric patient.

### DETAILED DESCRIPTION

The present disclosure relates to compositions comprising polyethylene glycol and inositol for the treatment of constipation and related conditions. For example, a disclosed composition when administered to a patient, can be effective in relieving constipation in a patient in about one-third the time, about half the time, less than one-third the time, or less than one half the time as compared to administering a composition having polyethylene glycol and no inositol.

Unless defined otherwise, all technical and scientific terms used herein have the same meaning as commonly understood by one of ordinary skill in the art to which this disclosure pertains.

Throughout the description, where compositions and kits are described as having, including, or comprising specific components, or where processes and methods are described as having, including, or comprising specific steps, it is contemplated that, additionally, there are compositions and kits of the present disclosure that consist essentially of, or consist of, the recited components, and that there are processes and methods according to the present disclosure that consist essentially of, or consist of, the recited processing steps.

In the application, where an element or component is said to be included in and/or selected from a list of recited elements or components, it should be understood that the element or component can be any one of the recited elements or components, or the element or component can be selected from a group consisting of two or more of the recited elements or components.

Further, it should be understood that elements and/or features of a composition or a method described herein can be combined in a variety of ways without departing from the spirit and scope of the present disclosure, whether explicit or implicit herein. For example, where reference is made to a particular compound, that compound can be used in various embodiments of compositions of the present disclosure and/or in methods of the present disclosure, unless otherwise understood from the context. In other words, within this application, embodiments have been described and depicted in a way that enables a clear and concise application to be written and drawn, but it is intended and will be appreciated that embodiments can be variously combined or separated without parting from the present teachings and disclosure(s). For example, it will be appreciated that all features described and depicted herein can be applicable to all aspects of the disclosure(s) described and depicted herein.

The articles "a" and "an" are used in this disclosure to refer to one or more than one (i.e., to at least one) of the grammatical object of the article, unless the context is inappropriate. By way of example, "an element" means one element or more than one element.

The term "and/or" is used in this disclosure to mean either "and" or "or" unless indicated otherwise.

It should be understood that the expression "at least one of' includes individually each of the recited objects after the expression and the various combinations of two or more of the recited objects unless otherwise understood from the context and use. The expression "and/or" in connection with three or more recited objects should be understood to have the same meaning unless otherwise understood from the context.

The use of the term "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

Where the use of the term "about" is before a quantitative value, the present disclosure also include the specific quantitative value itself, unless specifically stated otherwise. As used herein, the term "about" refers to a ±10% variation from the nominal value unless otherwise indicated or inferred.

Where a percentage is provided with respect to an amount of a component or material in a composition, the percentage should be understood to be a percentage based on weight, unless otherwise stated or understood from the context.

Where a molecular weight is provided and not an absolute value, for example, of a polymer, then the molecular weight should be understood to be an average molecule weight, unless otherwise stated or understood from the context.

It should be understood that the order of steps or order for performing certain actions is immaterial so long as the present disclosure remain operable. Moreover, two or more steps or actions can be conducted simultaneously.

At various places in the present specification, variables are disclosed in groups or in ranges. It is specifically intended that the description include each and every individual subcombination of the members of such groups and ranges. For example, an integer in the range of 0 to 40 is specifically intended to individually disclose 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40, and an integer in the range of 1 to 20 is specifically intended to individually disclose 1, 2, 3, 4, 5, 6, 7, 8, 9, 10, 11, 12, 13, 14, 15, 16, 17, 18, 19, and 20.

The phrases "pharmaceutically acceptable," as used herein, refers to compounds, molecular entities, compositions, materials, and/or dosage forms that do not produce an adverse, allergic, or other untoward reaction when administered to an animal, or a human, as appropriate.

The phrases "pharmaceutically acceptable carrier" and "pharmaceutically acceptable excipient," as used herein, refer to any and all solvents, dispersion media, coatings, isotonic and absorption delaying agents, and the like, that are compatible with pharmaceutical administration. Pharmaceutically acceptable carriers can include phosphate buffered saline solution, water, emulsions (e.g., such as an oil/water or water/oil emulsions), and various types of wetting agents. The compositions also can include stabilizers and preservatives.

The phrase "therapeutically effective amount," as used herein, refers to the amount of a composition (e.g., a disclosed composition) that will elicit the biological or medical response of a tissue, system, animal or human that is being sought by the researcher, veterinarian, medical doctor or other clinician. The compositions described in the disclosure can be administered in therapeutically effective amounts to treat a disease or disorder (e.g., IBS), a symptom of a disease or disorder (e.g., constipation, abdominal pain), and/or to elicit a desired biological response in a patient (e.g., to induce colon or bowel cleansing in a patient). A therapeutically effective amount of a composition can be the quantity required to achieve a desired therapeutic and/or a prophylactic effect. A therapeutically effective amount of a composition disclosed herein can be an amount which results in lessening of a symptom of a disease, such as lessening the amount, severity, and/or degree of constipation associated with a disease, disorder, or condition, for example, constipation associated with IBS.

The use of any and all examples, or exemplary language herein, for example, "such as" or "including," is intended merely to illustrate better the present disclosure and does not pose a limitation on the scope of the disclosure unless claimed. No language in the specification should be construed as indicating any non-claimed element as essential to the practice of the present disclosure.

Various aspects of the disclosure are set forth herein under headings and/or in sections for clarity; however, it is understood that all aspects, embodiments, or features of the disclosure described in one particular section are not to be limited to that particular section but rather can apply to any aspect, embodiment, or feature of the present disclosure.

### Compositions

The present disclosure provides a composition comprising polyethylene glycol, inositol, and one or more pharmaceutically acceptable excipients. In one aspect, a dry composition comprising polyethylene glycol, inositol, and one or more pharmaceutically acceptable excipients for the treatment of constipation is provided. In another aspect, a composition comprising polyethylene glycol, inositol, and one or more pharmaceutically acceptable excipients for colon or bowel cleansing is provided. In yet another aspect, a composition comprising polyethylene glycol, inositol, and one or more pharmaceutically acceptable excipients for the treatment of irritable bowel syndrome with constipation is provided. In yet a further aspect, a composition comprising polyethylene glycol, inositol, and one or more pharmaceutically acceptable excipients for the treatment of abdominal pain and/or constipation is provided. The compositions described herein may be for use in therapy, more specifically for use in the treatment of constipation, for use in colon or bowel cleansing, for use in the treatment of irritable bowel syndrome with constipation or for use in the treatment of abdominal pain and/or constipation. Furthermore, the use of the compositions described herein in the manufacture of medicaments for use in the treatment of constipation, for use in colon or bowel cleansing, for use in the treatment of irritable bowel syndrome with constipation or for use in the treatment of abdominal pain and/or constipation is provided herein.

Compositions include polyethylene glycol, for example, macrogol 4000. Macrogol is the International Nonproprietary Name (INN) for polyethylene glycol (alternately referred to herein as "PEG"). In some embodiments, polyethylene glycol may be macrogol 4000, macrogol 3350, macrogol 6000, or a mixture of two or more macrogols. A composition disclosed herein may include one or more of PEG 200, PEG 300, PEG 400, PEG 600, PEG 1000, PEG 1450, PEG 1500, PEG 2000, PEG 3000, PEG 4600, PEG 8000, PEG 10,000, or PEG 12,000.

In some embodiments, a composition comprises between about 95% to about 98% by weight polyethylene glycol. In some embodiments, a composition comprises between about 94% to about 98%, between about 95% to about 99%, between about 96% to about 98%, between about 96% to about 99%, between about 95% to about 99%, between about 94% to about 99%, between about 93% to about 98%, between about 92% to about 98%, between about 91% to about 98%, between about 90% to about 98%, between about 90% to about 99%, about 94%, about 94.5%, about 95%, about 95.5%, about 96%, about 96.5%, about 97%, about 97.5%, about 98%, about 98.5%, or about 99% by weight polyethylene glycol.

Exemplary compositions include dosage forms that include between about 500 mg to about 20 g of polyethylene glycol. For example, compositions can include about 500 mg, about 600 mg, about 700 mg, about 800 mg, about 900 mg, about 1 g, about 2 g, about 3 g, about 4 g, about 5 g, about 6 g, about 7 g, about 8 g, about 9 g, about 10 g, about 11 g, about 12 g, about 13 g, about 14 g, about 15 g, about 16 g, about 17 g, about 18 g, about 19 g, or about 20 g, between about 500 mg to about 1 g, between about 1 g to about 2 g, between about 2 g to about 3 g, between about 3 g to about 4 g, between about 4 g to about 5 g, between about 5 g to about 6 g, between about 6 g to about 7 g, between about 7 g to about 8 g, between about 8 g to about 9 g, between about 9 g to about 10 g, between about 10 g to about 11 g, between about 11 g to about 12 g, between about 12 g to about 13 g, between about 13 g to about 14 g, between about 14 g to about 15 g, between about 15 g to about 16 g, between about 16 g to about 17 g, between about 17 g to about 18 g, between about 18 g to about 19 g, between about 19 g to about 20 g, between about 500 mg to about 5 g, between about 1 g to about 5 g, between about 1 g to about 10 g, between about 5 g to about 10 g, between about 10 g to about 15 g, or between about 15 g to about 20 g of polyethylene glycol.

Inositol, or cyclohexane-1,2,3,4,5,6-hexol, is a sugar alcohol which exists in 9 possible stereoisomers (myo-inositol, scyllo-inositol, muco-inositol, D-chiro-inositol, neo-inositol, L-chiro-inositol, allo-inositol, epi-inositol, and cis-inositol). For example, *cis-*1,2,3,5-*trans*-4,6-cyclohexanehexol is commonly called myo-inositol. In various embodiments, a composition includes myo-inositol, or an isomer of myo-inositol, or myo-inositol and one or more isomers thereof.

In some embodiments, a composition includes between about 0.01% to about 2% by weight inositol. In some embodiments, a composition comprises about 0.5% by weight inositol. For example, in some embodiments a composition comprises about 0.01%, about 0.02%, about 0.03%, about 0.04%, about 0.05%, about 0.06%, about 0.07%, about 0.08%, about 0.09%, about 0.1%, about 0.2%, about 0.3%, about 0.35%, about 0.4%, about 0.41%, about 0.42%, about 0.43%, about 0.44%, about 0.45%, about 0.46%, about 0.47%, about 0.48%, about 0.49%, about 0.51%, about 0.52%, about 0.53%, about 0.54%, about 0.55%, about 0.56%, about 0.57%, about 0.58%, about 0.59%, about 0.6%, about 0.65%, about 0.7%, about 0.8%, about 0.9%, about 1%, between about 0.01% to about 0.05%, between about 0.01% to about 0.1%, between about 0.05% to about 1%, between about 0.1% to about 1%, between about 0.2% to about 0.9%, between about 0.3% to about 0.8%, between about 0.4% to about 0.6%, between about 1% to about 2%, or between about 1% to about 1.5% by weight inositol.

In certain embodiments, compositions including polyethylene glycol in combination with inositol, when administered to a patient, may provide an additive or synergistic therapeutic effect, e.g., may result in smooth muscle cell contraction in the gut and/or increased osmotic action, resulting in a faster effect on constipation. For example, disclosed compositions, when administered to a patient, can be effective in relieving constipation in a patient in about one-third the time, about half the time, less than one-third the time, or less than half the time as compared to administering a composition having polyethylene glycol and no inositol.

Compositions of the disclosure may include one or more pharmaceutically acceptable excipients, such as soy lecithin. In certain embodiments, a composition comprises between about 0.001% to about 0.1% by weight soy lecithin, e.g., comprises about 0.01% by weight soy lecithin, about 0.001%, about 0.002%, about 0.003%, about 0.004%, about 0.005%, about 0.006%, about 0.007%, about 0.008%, about 0.009%, about 0.0095%, about 0.01%, about 0.015%, about 0.02%, about 0.025%, about 0.03%, about 0.04%, or about 0.05%, about 0.06%, about 0.07%, about 0.08%, about 0.09%, about 0.1%, between about 0.001% to about 0.1%, between about 0.005% to about 0.05%, between about 0.005% to about 0.025%, between about 0.005% to about 0.07%, between about 0.001% to about 0.01%, between about 0.005% to about 0.01%, between about 0.01% to about 0.05%, between about 0.05% to about 0.1%, or between about 0.01% to about 0.01% by weight soy lecithin.

A composition may include a pharmaceutically acceptable excipient such as citric acid, alone or in addition to other excipients. For example, a composition may include about 2% by weight citric acid, or about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2.1%, about 2.2%, about 2.3%, about 2.4%, about 2.5%, about 2.6%, about 2.7%, about 2.8%, about 2.9%, about 3%, about 3.5%, or about 4%, about 5%, about 6%, about 7%, about 8%, about 9%, about 10%, between about 0.01% to about 10%, between about 0.05% to about 5%, between about 1% to about 5%, between about 1% to about 2%, between about 1% to about 3%, between about 1.5% to about 2.5%, between about 1.5% to about 2%, between about 2% to about 2.5%, between about 2% to about 3%, or between about 0.05% to about 5% by weight citric acid.

Pharmaceutically acceptable excipients useful in the compositions of the disclosure may include one or more of: magnesium citrate, calcium citrate, acesulfame potassium, potassium chloride, mannitol, gluconolactone, sorbitol, anhydrous silica colloidal, sodium bicarbonate, sodium hydrogen carbonate, sodium chloride, sodium sulfate, magnesium sulfate, and tricalcium phosphate.

In various embodiments, a pharmaceutically acceptable excipient may include a flavouring agent (e.g., orange flavor, strawberry flavor, cherry flavor, or a lemon flavouring agent) and/or sweetener. For example, a composition may include about 1.6% by weight flavouring agent, or about 0.5%, about 0.6%, about 0.7%, about 0.8%, about 0.9%, about 1%, about 1.1%, about 1.2%, about 1.3%, about 1.4%, about 1.5%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, between about 0.5% to about 2%, between about 0.5% to about 3%, between about 1% to about 2%, or between about 1% to about 3% by weight flavouring agent (e.g., orange flavor, strawberry flavor, cherry flavor, or lemon flavouring agent).

In some embodiments, a disclosed composition is provided as a powder composition. In certain embodiments, a liquid composition having the components as described herein may also be provided. For example, a dry composition of the disclosure can be reconstituted before administration, for example, a liquid composition may include water and a dry composition such as disclosed herein. For example, a liquid composition for oral administration may comprise about 100 mL water and a disclosed dry composition comprising about 10 g polyethylene glycol. In various embodiments, a liquid composition may comprise about 50 mL, about 75 mL, about 125 mL, about 150 mL, about 175 mL, about 200 mL, between about 50 mL to about 200 mL, between about 50mL to about 175mL, or between about 75 mL to about 175 mL water and a disclosed dry composition comprising about 5 g, about 7.5 g, about 12.5 g, about 15 g, about 20 g, between about 5 g to about 20 g, between about 7.5 g to about 15 g, between about 7.5 g to about 12.5 g of polyethylene glycol.

### Unit dosage form

A "unit dosage form" as used herein refers to a physically discrete unit suited as unitary dosage for a patient to be treated. For example, each unit dosage form can contain a predetermined quantity of a composition comprising polyethylene glycol, inositol, and, optionally, one or more pharmaceutically acceptable excipients, calculated to produce a desired therapeutic effect. A unit dosage form can be in the form of a tablet, or a dry powder in a container such as a sachet, vial or a water-soluble packet. A unit dosage form can be in the form of a liquid in a container; however, when a liquid form of the compositions disclosed herein is administered, a dry composition is usually dissolved or mixed with an appropriate liquid carrier such as water soon before use.

Provided herein is a unit dosage form for the treatment of constipation comprising polyethylene glycol, inositol, and one or more pharmaceutically acceptable excipients. Also provided herein is a unit dosage form for the treatment of irritable bowel syndrome (IBS), abdominal pain, and/or constipation, or for colon or bowel cleansing, comprising a composition disclosed herein. The unit dosage forms described herein may also be for use in therapy, more specifically for use in the treatment of constipation, or for use in the treatment of irritable bowel syndrome (IBS), abdominal pain, and/or constipation, or for use in colon or bowel cleansing.

In some embodiments, a unit dosage form comprises about 10 g of polyethylene glycol and between about 1 mg to about 250 mg (e.g., between about 1 mg to about 60 mg, between about 20 mg to about 40 mg, between about 30 mg to about 60 mg, or between about 20 mg to 60 mg) of inositol. For example, a unit dosage form may comprise about 5 g of polyethylene glycol and between about 0.5 mg to about 125 mg of inositol, about 15 g of polyethylene glycol and between about 1.5 mg to about 375 mg of inositol, about 20 g of polyethylene glycol and between about 2 mg to about 500 mg of inositol, between about 5 g to about 10 g of polyethylene glycol and between 0.5 mg to about 250 mg of inositol, between about 5 g to about 15 g of polyethylene glycol and between about 0.5 mg to about 375 mg (e.g., between about 1 mg to about 60 mg, between about 20 mg to about 40 mg, between about 30 mg to about 60 mg, or between about 20 mg to 60 mg) of inositol, or between about 5 g to about 20 g of polyethylene glycol and between about 0.5 mg to about 500 mg of inositol.

Amounts of a disclosed composition as described herein in a unit dosage form may vary according to factors such as the disease state, age, sex, and weight of the individual patient. Dosage regimens may be adjusted to provide the optimum therapeutic response in the patient. For example, a single dose may be administered, several divided doses may be administered over time, or the dose may be proportionally reduced or increased as indicated by the exigencies of the therapeutic situation. It is especially advantageous to formulate compositions in a unit dosage form for ease of administration and uniformity of dosage.

### Methods

Also provided herein is a method of treating constipation comprising administering to a patient in need thereof, a composition comprising polyethylene glycol, inositol, and one or more pharmaceutically acceptable excipients. Also contemplated herein is a method of treating constipation comprising a composition disclosed herein. Also provided herein is a method of treating constipation comprising dissolving a disclosed dry composition in water, and administering the final dissolved composition orally to a patient in need thereof.

In some aspects, a method of treating constipation comprises administering to a patient in need thereof a unit dosage form disclosed herein. In some embodiments, said unit dosage form comprises about 10 g of polyethylene glycol and between about 1 mg to about 250 mg (e.g., between about 1 mg to about 60 mg, between about 20 mg to about 40 mg, between about 30 mg to about 60 mg, or between about 20 mg to 60 mg) of inositol. For example, a unit dosage form may comprise about 5 g of polyethylene glycol and between about 0.5 mg to about 125 mg of inositol, about 15 g of polyethylene glycol and between about 1.5 mg to about 375 mg of inositol, about 20 g of polyethylene glycol and between about 2 mg to about 500 mg of inositol, between about 5 g to about 10 g of polyethylene glycol and between 0.5 mg to about 250 mg of inositol, between about 5 g to about 15 g of polyethylene glycol and between about 0.5 mg to about 375 mg (e.g., between about 1 mg to about 60 mg, between about 20 mg to about 40 mg, between about 30 mg to about 60 mg, or between about 20 mg to 60 mg) of inositol, or between about 5 g to about 20 g of polyethylene glycol and between about 0.5 mg to about 500 mg of inositol.

Methods of the disclosure may include administering a disclosed composition once per week, twice per week, three times per week, four times per week, five times per week, six times per week, once a day, twice a day, three times a day, four times a day, or five times a day. For example, a composition may be administered once per day. In some embodiments, a composition comprising half of a dosage suitable for an adult patient may be administered to a pediatric patient.

In certain embodiments, a method of colon or bowel cleansing is provided, the method generally comprising administering a disclosed composition to a patient in need thereof. For example, colon or bowel cleansing can be done prior to colonoscopy and barium enema radiologic examinations.

In some embodiments, a method of treating irritable bowel syndrome (IBS) with constipation comprises administering a disclosed composition to a patient in need thereof. IBS is a common disorder that affects the large intestine (colon) and commonly causes cramping, abdominal pain, bloating, gas, diarrhea, and constipation. In certain embodiments, a method for treating abdominal pain and/or constipation comprises administering a disclosed composition to a patient in need thereof.

In various embodiments, a method for treating chronic constipation comprises administering a disclosed composition to a patient in need thereof. In some embodiments, a method for treating acute constipation comprises administering a disclosed composition to a patient in need thereof. Also provided herein, is a method for treating opioid-induced constipation comprising administering a disclosed composition to a patient in need thereof.

Efficacy of treatment may be evaluated based on stool frequency and/or consistency. In some embodiments, a method of treating constipation comprises administering a disclosed composition to a patient in need thereof, wherein the constipation is evaluated based on stool frequency and/or consistency. For example, a method of treating constipation, wherein the method is effective to increase stool frequency at least once per week as compared with a baseline, is provided. In other examples, a method of treating constipation comprises administering a disclosed composition to a patient in need thereof, wherein the method is effective to increase stool frequency at least twice per week, three times per week, four times per week, five times per week, or six times per week as compared with a baseline.

Efficacy of treatment may also be evaluated based on changes in intensity of abdominal pain or frequency of complete spontaneous bowel movements (CSBM). In various embodiments, a method of treating abdominal pain and/or constipation comprises administering a disclosed composition to a patient in need thereof, wherein said abdominal pain and/or constipation is evaluated based on changes in intensity of abdominal pain and/or frequency of CSBM. For example, a method of treating abdominal pain and/or constipation can include administering a disclosed composition to a patient in need thereof, wherein the method is effective to a) decrease the intensity of said abdominal pain by 30 percent or more based on the weekly average of worst abdominal pain score or the average of the worst abdominal pain score in the previous 24 hours score as compared with a baseline pain score; and/or b) increase the number of complete spontaneous bowel movements (CSBM) per week to at least one more per week as compared with a baseline. For example, a method of treating abdominal pain and/or constipation can include administering a disclosed composition to a patient in need thereof, wherein the method is effective to a) decrease the intensity of said abdominal pain by 10 percent or more, 20 percent or more, 40 percent or more, 50 percent or more, 60 percent or more, 70 percent or more, 80 percent or more, 90 percent or more, or 100 percent or more, based on the weekly average of worst abdominal pain score or the average of the worst abdominal pain score in the previous 24 hours score as compared with a baseline score; and/or b) increase the number of complete spontaneous bowel movements (CSBM) per week to at least two more times per week, three more times per week, four more times per week, five more times per week, or six more times per week as compared with a baseline.

### Kits

In various embodiments, the disclosure features a kit for the treatment of constipation. A kit can include a composition comprising polyethylene glycol, inositol, and one or more pharmaceutically acceptable excipients. In some embodiments, a kit for the treatment of constipation comprises a composition disclosed herein. In certain embodiments, a kit for colon or bowel cleansing is provided, which comprises a disclosed composition is provided. In particular embodiments, a kit for the treatment of IBS or abdominal pain and/or constipation comprising a disclosed composition is provided.

In certain embodiments, a kit for the treatment of constipation comprises a unit dosage form, and, optionally, instructions for use, and/or a container, e.g., a container suitable for mixing a disclosed dry dosage form with a suitable liquid such as water for constitution into a liquid composition. Also provided herein is a kit for the treatment of irritable bowel syndrome, abdominal pain, and/or constipation, or for colon or bowel cleansing, each comprising a unit dosage form disclosed herein. In some embodiments, a unit dosage form comprises about 10 g of polyethylene glycol and between about 1 mg to about 250 mg (e.g., between about 1 mg to about 60 mg, between about 20 mg to about 40 mg, between about 30 mg to about 60 mg, or between about 20 mg to 60 mg) of inositol. For example, a unit dosage form may comprise about 5 g of polyethylene glycol and between about 0.5 mg to about 125 mg of inositol, about 15 g of polyethylene glycol and between about 1.5 mg to about 375 mg of inositol, about 20 g of polyethylene glycol and between about 2 mg to about 500 mg of inositol, between about 5 g to about 10 g of polyethylene glycol and between 0.5 mg to about 250 mg of inositol, between about 5 g to about 15 g of polyethylene glycol and between about 0.5 mg to about 375 mg (e.g., between about 1 mg to about 60 mg, between about 20 mg to about 40 mg, between about 30 mg to about 60 mg, or between about 20 mg to 60 mg) of inositol, or between about 5 g to about 20 g of polyethylene glycol and between about 0.5 mg to about 500 mg of inositol.

In certain embodiments, a kit includes a single-dose preparation, for example, a unit dosage form for treating irritable bowel syndrome, abdominal pain, and/or constipation, or for colon or bowel cleansing. Kits may include a container. In some embodiments, a kit may comprise instructions for use, for example, in treating constipation. A kit may include 2 sachets, for example, where together the two sachets provide a suitable adult dosage and /or 1 sachet provides a suitable pediatric dosage.

### Treatment

A "patient," as described herein, refers to any animal at risk for, suffering from or diagnosed for constipation, including, but not limited to, mammals, primates, and humans. In certain embodiments, the patient may be a non-human mammal such as, for example, a cat, a dog, or a horse.

"A patient in need," as used herein, refers to a patient suffering from any of the symptoms or manifestations of constipation, a patient who may suffer from any of the symptoms or constipation, or any patient who might benefit from a method of the disclosure for treating constipation.

The terms "treat", "treatment", "treating" and the like are used herein to generally mean obtaining a desired pharmacological and/or physiological effect. The effect may be prophylactic in terms of completely or partially preventing constipation or symptoms thereof and/or may be therapeutic in terms of partially or completely curing constipation and/or adverse effects attributed to constipation. The term "treatment" as used herein covers any treatment of constipation in a mammal, particularly a human, and includes: (a) preventing constipation from occurring in a subject which may be predisposed to constipation but has not yet been diagnosed as having it; (b) inhibiting constipation, for example, preventing constipation from increasing in severity or scope; (c) relieving constipation, for example, causing partial or complete amelioration of constipation; or (d) preventing relapse of constipation, for example, preventing constipation from returning to an active state following previous successful treatment of symptoms of constipation or treatment of constipation.

"Constipation" is a condition in which there is difficulty in emptying the bowels, usually associated with hardened feces. For example, constipation may be chronic constipation, acute constipation, or opioid-induced constipation. Some of the symptoms of constipation includes, but not limited to, lower abdominal discomfort, infrequent bowel movements, straining to have a bowel movement, hard or small stools, rectal bleeding and/or anal fissures caused by hard stools, and physiological distress and/or obsession with having bowel movements.

### EXAMPLES

### Example 1: Powder Formulation A in Sachets

The components listed in Table 1 are mixed together to form formulation A, which can be used in the treatment of constipation. After mixing, an amount of formulation A is loaded in a sachet.

**Table 1. Formulation A**

| **Component** | **% w/w** |
|---|---|
| Macrogol 4000 | 95.89 |
| Citric acid | 2.00 |
| Orange flavor | 1.60 |
| Inositol | 0.50 |
| Soy lecithin | 0.01 |
| **TOTAL** | **100.00** |

### Example 2: Powder Formulation B in Sachets

The components listed in Table 2 are mixed together to form formulation B, which can be used in the treatment of constipation. After mixing, an amount of formulation B is loaded in a sachet.

**Table 2. Formulation B**

| **Component** | **% w/w** |
|---|---|
| Macrogol 4000 | 97.89 |
| Strawberry flavor | 1.60 |
| Inositol | 0.50 |
| Soy lecithin | 0.01 |
| **TOTAL** | **100.00** |

### INCORPORATION BY REFERENCE

The entire disclosure of each of the patent documents and scientific articles cited herein is incorporated by reference for all purposes.

### EQUIVALENTS

The disclosure can be embodied in other specific forms with departing from the essential characteristics thereof. The foregoing embodiments therefore are to be considered illustrative rather than limiting on the disclosure described herein. The scope of the disclosure is indicated by the appended claims rather than by the foregoing description, and all changes that come within the meaning and range of equivalency of the claims are intended to be embraced therein.

The present invention further relates to the following numbered clauses.
1. A dry composition for the treatment of constipation, the dry composition comprising:
   polyethylene glycol;
   inositol; and
   one or more pharmaceutically acceptable excipients.
2. The composition of clause 1, wherein said polyethylene glycol is selected from the group consisting of macrogol 4000, macrogol 3350, macrogol 6000, and mixtures thereof.
3. The composition of clause 1 or 2, wherein the polyethylene glycol is macrogol 4000.
4. The composition of any one of clauses 1-3, wherein the composition comprises about 90% to about 98% by weight polyethylene glycol.
5. The composition of clause 4, wherein the composition comprises about 95% to about 98 % by weight polyethylene glycol.
6. The composition of clause 4, wherein the composition comprises about 96% by weight polyethylene glycol.
7. The composition of clause 4, wherein the composition comprises about 98% by weight polyethylene glycol.
8. The composition of any one of clauses 1-7, wherein the composition comprises between about 0.01% to about 2% by weight inositol.
9. The composition of clause 8, wherein the composition comprises about 0.5% by weight inositol.
10. The composition of any one of clauses 1-9, wherein said inositol is myo-inositol, or a mixture of myo-inositol and one or more isomers thereof.
11. The composition of any one of clauses 1-10, wherein the pharmaceutically acceptable excipient comprises soy lecithin.
12. The composition of clause 11, wherein the composition comprises between about 0.001% to about 0.1% by weight soy lecithin.
13. The composition of clause 12, wherein the composition comprises about 0.01% by weight soy lecithin.
14. The composition of any one of clauses 1-13, wherein said pharmaceutically acceptable excipient comprises citric acid.
15. A liquid composition for oral administration, the liquid composition comprising:
   the composition of any one of clauses 1-14; and
   water.
16. A unit dosage form for the treatment of constipation, the unit dosage form comprising:
   polyethylene glycol;
   inositol; and
   one or more pharmaceutically acceptable excipients.
17. A method of treating constipation, the method comprising administering to a patient in need thereof, the composition of any one of clauses 1-15.
18. A method of treating constipation, the method comprising:
   dissolving the dry composition of any one of clauses 1-15 in water to provide a dissolved composition; and
   administering the dissolved composition orally to a patient in need thereof.
19. A kit for the treatment of constipation, the kit comprising:
   the composition of any one of clauses 1-15; and
   instructions for the use of the composition.
20. A kit for the treatment of constipation, the kit comprising the unit dosage form of clause 16.
21. The kit of clause 19 or 20, wherein the kit further comprises a container.
22. The kit of any one of clauses 19-21, wherein the kit comprises 2 sachets that include about 10 g of polyethylene glycol and between about 1 mg to about 60 mg of inositol, and instructions indicating 2 sachets are suitable for treating an adult patient and 1 sachet is suitable to treat a pediatric patient.
23. A dry composition of clause 1, wherein the dry composition comprises: about 96 % w/w macrogol 4000, about 2% w/w citric acid, about 1.6% w/w orange flavoring agent, about 0.5% w/w inositol, and about 0.01% w/w soy lecithin.
24. A dry composition of clause 1, the composition comprising: about 98 % w/w macrogol 4000, about 1.6% w/w strawberry flavoring agent, about 0.5% w/w inositol, and about 0.01% w/w soy lecithin.
25. The dry composition of any one of clauses 1-14, 23, or 24, wherein the dry composition is a pharmaceutically acceptable composition.

## Claims

1. A dry composition for the treatment of constipation, the dry composition comprising:
polyethylene glycol;
inositol; and
one or more pharmaceutically acceptable excipients.

2. The composition of claim 1, wherein said polyethylene glycol is selected from the group consisting of macrogol 4000, macrogol 3350, macrogol 6000, and mixtures thereof.

3. The composition of claim 1 or 2, wherein the composition comprises about 90% to about 98% by weight polyethylene glycol.

4. The composition of claim 3, wherein the composition comprises:
(a) about 95% to about 98 % by weight polyethylene glycol; or
(b) about 96% by weight polyethylene glycol; or
(c) about 98% by weight polyethylene glycol.

5. The composition of any one of claims 1-4, wherein:
(a) the composition comprises between about 0.01% to about 2% by weight inositol; and/or
(b) said inositol is myo-inositol, or a mixture of myo-inositol and one or more isomers thereof; and/or
(c) the one or more pharmaceutically acceptable excipients comprises soy lecithin; and/or
(d) said one ore more pharmaceutically acceptable excipients comprises citric acid.

6. The composition of claim 5 part (a), wherein the composition comprises about 0.5% by weight inositol.

7. The composition of claim 5 part (c), wherein the composition comprises between about 0.001% to about 0.1% by weight soy lecithin, optionally about 0.01% by weight soy lecithin.

8. A liquid composition for oral administration, the liquid composition comprising:
the composition of any one of claims 1-7; and
water.

9. A unit dosage form for the treatment of constipation, the unit dosage form comprising:
polyethylene glycol;
inositol; and
one or more pharmaceutically acceptable excipients.

10. The composition of any one of claims 1-8 for use in a method of treating constipation, the method comprising administering said composition to a patient in need thereof..

11. The dry composition of any one of claims 1-7 for use in a method of treating constipation, the method comprising:
dissolving said dry composition in water to provide a dissolved composition; and
administering the dissolved composition orally to a patient in need thereof.

12. A kit for the treatment of constipation, the kit comprising:
(a) the composition of any one of claims 1-8; and
instructions for the use of the composition; or
(b) the unit dosage form of claim 9;
optionally wherein the kit further comprises a container.

13. The kit of claim 12, wherein the kit comprises 2 sachets that include about 10 g of polyethylene glycol and between about 1 mg to about 60 mg of inositol, and instructions indicating 2 sachets are suitable for treating an adult patient and 1 sachet is suitable to treat a pediatric patient.

14. A dry composition of claim 1, wherein the dry composition comprises:
(a) about 96 % w/w macrogol 4000, about 2% w/w citric acid, about 1.6% w/w orange flavoring agent, about 0.5% w/w inositol, and about 0.01% w/w soy lecithin; or
(b) about 98 % w/w macrogol 4000, about 1.6% w/w strawberry flavoring agent, about 0.5% w/w inositol, and about 0.01% w/w soy lecithin.

15. The dry composition of any one of claims 1-7 or 14, wherein the dry composition is a pharmaceutically acceptable composition.
